# FASCICULE DE BREVET EUROPEEN

(11) **EP 0 516 817 B1**
(45) Date de publication et mention de la délivrance du brevet: **06.03.1996**
(21) Numéro de dépôt: 92903122.7
(22) Date de dépôt: 26.12.1991
(51) Int. Cl.: C12Q 1/10

(54) **PROCEDE ET MILIEU DE DETECTION DES SALMONELLA**
VERFAHREN UND MEDIUM ZUM NACHWEIS VON SALMONELLEN
METHOD AND MEDIUM FOR DETECTING SALMONELLA

(30) Priorité: 28.12.1990 FR 9016635
(43) Date de publication de la demande: 09.12.1992
(73) Titulaire: BIO MERIEUX, Société anonyme, F-69280 Marcy l'Etoile (FR)
(72) Inventeur: MONGET, Daniel, F-01150 Saint-Sorlin-en-Bugey (FR); VILLEVAL, François, F-69190 Saint-Genis-les-Ollières (FR)
(74) Mandataire: Guerre, Dominique
(86) Numéro de dépôt international: FR9101075
(87) Numéro de publication internationale: WO9212259

(56) Documents cités:
- EP-A- 0 214 340
- EP-A- 0 282 733
- US-A- 3 856 628
- US-A- 4 591 554
- APPLIED & ENVIRONMENTAL MICROBIOLOGY, vol. 56, no. 1, 01 janvier 1990, Washington, DC (US); RAMBACH, pp. 301-303/
- WPIL, Week 9117, 12 juin 1991, Derwent Publications Ltd., London (GB); AN 91123670/
- WPIL, Week 8621, Derwent Publications Ltd., London (GB); AN 86-132750/

## Description

La présente invention concerne la caractérisation ou l'isolement des bactéries du genre Salmonella, par ensemencement d'un milieu de culture spécifique avec un inoculum présumé contenir lesdites bactéries, croissance de ces dernières sur le milieu de culture, et développement d'un ou plusieurs phénomènes chromogènes ou fluorigènes de caractérisation, à partir de un ou plusieurs caractères biochimiques propres aux bactéries recherchées.

Les bactéries Salmonella sont des entérobactéries à Gram négatif, présentant de manière générale les caractères biochimiques suivants :
- lactose -
- ONPG -
- uréase -
- H₂S +

Il n'existe pas de caractère biochimique attribuable uniquement au genre Salmonella, de telle sorte que la caractérisation de ces bactéries passe en général par la mise en oeuvre d'une pluralité, voire un grand nombre de tests biochimiques, complétés éventuellement par des tests immunologiques ou par des examens morphologiques.

Il existe cependant dans le commerce des milieux gélosés, présentés comme étant sélectifs des Salmonella, et mettant en oeuvre au moins deux tests biochimiques, travaillant chacun par présence ou absence d'une couleur.

Ces milieux contiennent généralement des inhibiteurs des bactéries à Gram positif et de certaines bactéries à Gram négatif. Parmi les inhibiteurs couramment employés, on peut citer les sels biliaires (désoxycholate de sodium, etc...), le citrate de sodium, le vert brillant.

La détection des Salmonella sur ces milieux est possible par :
- l'absence de fermentation d'un ou plusieurs hydrates de carbone (lactose, etc...)
- la recherche de la production d'H₂S, caractère biochimique généralement présent chez Salmonella ; cette détection se fait en incorporant au milieu du thiosulfate de sodium et un sel ferrique.

Les principaux milieux gélosés utilisés pour la recherche des Salmonella sont :
- gélose D.C.L. de la Société Diagnostics Pasteur
- gélose D.C.L.S. des Sociétés BioMérieux et Diagnostics Pasteur
- gélose Hektoen des Sociétés BioMérieux et Diagnostics Pasteur
- gélose S.S. des Sociétés BioMérieux et Diagnostics Pasteur
- gélose Kristensen de la Société Diagnostics Pasteur
- gélose XLD des Sociétés BioMérieux et Diagnostics Pasteur.

Ces milieux ont cependant certaines limitations, liées au principe de caractérisation des Salmonella : toute colonie incolore (pas de fermentation d'hydrate de carbone) et/ou avec centre noir (production d'H₂S) doit être considérée comme suspecte.

Ces milieux ne sont en fait pas très spécifiques des Salmonella. En effet, certaines espèces peuvent se développer et donner des colonies présentant les mêmes caractéristiques que celles de Salmonella :
- colonies incolores (lactose (-)) :
   - Salmonella
   - Shigella
   - Serratia
   - Yersinia
   - Hafnia alvei
   - E. coli subsp. alkalescens dispar
   - Morganella morganii
   - Proteus rettgeri
- colonies à centre noir (H₂S (+)) :
   - Salmonella
   - Citrobacter
   - Proteus vulgaris
   - Proteus mirabilis

Par ailleurs, la production d'H₂S sur ces milieux n'est pas toujours constante. Ces variations peuvent être liées à différents facteurs, comme le pH, la concentration d'oxygène au niveau des colonies, etc...

Ainsi, bon nombre de colonies de Salmonella ne présentent pas le caractère positif H₂S et, de ce fait, n'ont pas de centre noir et restent incolores.

Il s'en suit un nombre important de confirmations inutiles par des "galeries d'identification biochimiques", et des sérums agglutinants, entraînant une perte de temps et grèvant le coût de l'examen.

Enfin, l'absence de coloration spécifique des Salmonella peut être une source d'erreurs. Il est en effet très facile de passer à côté d'une colonie suspecte. Cela est d'autant plus vrai lorsque les colonies sont incolores, ou présentent la même couleur que celle du milieu.

En d'autres termes, tous ces milieux n'apportent pas à eux seuls une fiabilité suffisante pour détecter les Salmonella, d'une part en raison des couleurs non distinctives retenues pour révéler la présence de ces bactéries, et d'autre part de la pluralité des phénomènes ou configurations colorés pouvant caractériser la présence de ces bactéries.

Conformément à la publication :

New plate medium for facilitated differentiation of Salmonella spp. from Proteus spp. and other enteric bacteria - Alain RAMBACH, Applied and Environmental Microbiology. Jan 1990, 56, p. 301-303
on a proposé un milieu de culture et de détection sélective de bactéries du genre Salmonella, comprenant :
- un substrat nutritif métabolisé par lesdites bactéries
- un composé chromogène susceptible d'être hydrolysé par l'enzyme β-galactosidase produit par la bactérie, pour développer une couleur, en l'occurrence le 5-bromo-4-chloro-3-indolyl-β-D-galactopyranoside, développant une couleur bleue, sous l'effet de son hydrolyse enzymatique par la β-galactosidase
- du propylène-glycol avec un indicateur de pH, tel que le rouge neutre.

Dans ce cas, le test biochimique principal utilisé pour détecter les Salmonella consiste en la fermentation acide par ces bactéries du propylène glycol, détectée par le rouge neutre. Et ce test principal est complété par celui de la β-galactosidase, cet enzyme étant absent chez les Salmonella, lesquelles n'hydrolysent donc pas le composé chromogène.

Au total, la grille des couleurs suivantes déterminerait l'interprétation ci-après :
- rouge brillant : présence de bactéries propylène-glycol + et β-galactosidase -, donc présence de Salmonella
- bleu : présence de bactéries propylène-glycol - et β-galactosidase +, donc absence de Salmonella
- violet : présence de bactéries propylène-glycol + et β-galactosidase +, donc absence de Salmonella
- incolore : présence de bactéries propylène-glycol - et β-galactosidase -, donc absence de Salmonella.

En pratique, des analyses bactériologiques effectuées par la Demanderesse avec le milieu de Rambach ont donné les résultats suivants, pour 33 souches ou espèces se répartissant en 12 Salmonella et 21 d'un genre autre que Salmonella :
- Salmonella (12) :
   + 3 souches (1 S. typhimurium, 1 S. paratyphi B, et 1 S. paratyphi C) ont développé la couleur rouge attendue,
   + 1 souche (S. arizonae) a développé une couleur bleue attendue,
   + 8 souches (4 S. typhi, 1 S. enterididis, 1 S. gallinarum, 1 S. pullorum et 1 S. paratyphi A) sont restées incolores,
- autres que Salmonella :
   + 1 souche (Pseudomonas aeruginosa) a développé une couleur rouge.

S'il est normal que les quatres souches de S. typhi demeurent incolores, comme déjà observé par Rambach, il est par contre anormal d'obtenir des tests négatifs pour cinq autres espèces de Salmonella, dont deux appartenant à des espèces vétérinaires (S. gallinarum et S. pullorum). Au surplus, une souche autre que Salmonella aboutit à un résultat positif.

En définitive, le milieu de détection proposé par Rambach apparaît être :
- non sélectif du genre Salmonella, puisque d'autres bactéries, notamment Pseudomonas aegurinosa, réagissent au même test
- sélectif de certaines souches ou espèces dans le genre Salmonella, puisqu'en particulier l'espèce S. typhi ne réagit pas au test proposé
- plus adapté aux Salmonella d'origine alimentaire ou vétérinaire, qu'à celles d'origine clinique.

Or, compte tenu des graves syndromes que sont capables de provoquer les Salmonella chez l'homme, dont les S. typhi responsables de la fièvre typhoïde, la mise au point d'un milieu permettant de distinguer pratiquement toutes les Salmonella représente aujourd'hui un réel besoin.

La présente invention vise à remédier aux inconvénients décrits précédemment.

Plus particulièrement, la présente invention a pour objet un milieu de caractérisation relativement fiable de la plupart des espèces Salmonella, rencontrées dans la pratique du contrôle bactériologique, avec un seuil de détection relativement faible.

La présente invention procède de la découverte selon laquelle :
- les espèces fermentant l'acide glucuronique et ne produisant pas de β-galactosidase sont en nombre très limité ; il s'agit de Salmonella (à l'exception de S. arizonae), de Morganella morganii, de Edwardsiella hoshinae, de Edwardsiella tarda, et Yersinia ruckeri
- ces trois dernières espèces sont très rarement isolées : aussi en pratique, la possibilité de les confondre avec une Salmonella est très faible
- en pratique, Morganella morganii peut être éliminée par l'addition d'inhibiteurs.

En conséquence, selon la présente invention, en associant au milieu de culture, en plus du composé chromogène ou fluorigène susceptible d'être hydrolysé par la β-galactosidase, l'acide glucuronique ou un sel de ce dernier, et un indicateur de pH, il est possible de détecter sélectivement les Salmonella, à l'exception de S. arizonae, directement sur isolement.

Par composé chromogène ou fluorigène, on entend tout marqueur dont la molécule, en présence d'au moins un enzyme spécifique, est susceptible d'être hydrolysée ou coupée en deux parties, à savoir une partie inerte, non chromogène ou fluorigène, et une partie chromogène ou fluorigène, pouvant développer une couleur visible à l'oeil nu, ou sous éclairement UV, directement, ou indirectement, c'est-à-dire par action d'un composé chimique complémentaire, dit révélateur.

L'efficacité du couple biochimique choisi selon la présente invention apparaît surprenante, en ce sens qu'à la différence de la plupart des hydrates de carbone auxquels il appartient, l'acide glucuronique n'inhibe pas la β-galactosidase, à l'exception de celle produite par les bactéries du genre Serratia qui, sur le milieu de l'invention, ne s'exprime pas.

Le milieu selon l'invention comprend un composé chromogénique ou fluorogénique, hydrolysable par la β-galactosidase qui peut être choisi parmi les composés p-nitrophényl-β-D-galactopyranoside, 4-méthylumbelliféryl-β-D-galactopyranoside et 5-bromo-4-chloro-3-indolyl-β-D-galactopyranoside.

Dans un mode de réalisation préféré du milieu selon l'invention, le composé chromogénique est le 5-bromo-4-chloro-3-indolyl-β-D-galactopyranoside, dont la teneur pondérale pour un litre de milieu est préférentiellement comprise entre 0,01 g et 1 g. Ce composé pigmente les colonies de bactéries productrices de β-galactosidase en bleu et présente l'avantage de limiter la coloration aux colonies sans diffusion dans le milieu.

Selon une composition particulière du milieu de l'invention, le milieu contient un inducteur de la β-galactosidase, qui peut être choisi parmi le lactose et l'isopropyl-β-D-thiogalactopyranoside.

La teneur pondérale préférentielle de l'isopropyl-β-D-thiogalactopyranoside est comprise entre 0 et 100 mg.

Le milieu selon l'invention peut contenir au moins un inhibiteur de croissance des bactéries n'appartenant pas à l'espèce des Salmonella. Cet inhibiteur peut être à la fois un activateur de croissance des Salmonella. Il peut être choisi parmi le vert brillant, le désoxycholate de sodium, et un mélange de sels biliaires. Les teneurs préférentielles du vert brillant et du désoxycholate de sodium, pour 1 l de milieu sont respectivement comprises entre 0 et 0,1 g et 0 à 10 g.

Le substrat nutritif du milieu selon l'invention est composé des ingrédients classiques indispensables au développement des bactéries. Selon un mode particulier de préparation du milieu de l'invention, il est constitué d'une peptone, d'un extrait de levure et de NaCI dont les teneurs pondérales pour 1 l de milieu sont préférentiellement comprises entre respectivement 1 et 20 g, 0 et 10 g, 0 et 10 g.

Selon un mode de réalisation particulier de l'invention, le milieu se présente sous forme solide gélosée, et le milieu comprend alors de l'agar dont la teneur pondérale pour 1 l de milieu est de préférence comprise entre 5 et 25 g.

Le milieu selon la présente invention contient de l'acide glucuronique ou un sel de ce dernier. Le milieu contient préférentiellement du glucuronate de sodium dont la teneur pondérale pour 1 l de milieu est de préférence comprise entre 1 et 30 g.

L'indicateur de pH contenu dans le milieu est choisi parmi le rouge de phénol, le bleu de bromothymol, le pourpre de bromocrésol et le rouge neutre. Dans une composition préférentielle du milieu selon l'invention, l'indicateur de pH est le rouge neutre, dont la teneur pondérale pour 1 l de milieu est de préférence comprise entre 5 et 100 mg.

Préférentiellement, le pH du milieu d'identification est compris entre 6 et 8.

Dans certains cas, on a observé qu'après 24 heures d'incubation d'un échantillon contenant des Salmonella sur le milieu de l'invention, la coloration spécifique obtenue pour les Salmonella peut évoluer, à cause d'une réalcalinisation du milieu. En particulier, quand l'indicateur de pH est le rouge neutre, la coloration rouge obtenue, caractéristique des caractères biochimiques :absence de β-galactosidase et fermentation de l'acide glucuronique ou de son sel, peut virer au jaune.

Pour améliorer le milieu de culture selon l'invention, on a trouvé qu'il convient d'ajouter au moins un sucre fermentescible par les Salmonella, ce sucre est choisi parmi le mélibiose, le sorbitol, le dulcitol, le mannitol, le glucose et le glucuronate et leurs mélanges, en quantité comprise entre 1 et 10 g par litre de milieu.

Selon une composition préférentielle du milieu de l'invention, ce dernier comprend du sorbitol, en quantité comprise entre 5 et 10 g par litre, avantageusement 8 g par litre.

Dans cette dernière composition, le sorbitol apporté au milieu peut faire apparaître les bactéries du genre Serratia comme faux positif.

En effet, ces bactéries qui ne fermentent pas l'acide glucuronique et produisent la β-galactosidase, non exprimée sur le milieu de l'invention en l'absence de sorbitol, restent donc incolores sur ledit milieu, mais elles se peuvent se colorer en rouge quand le milieu de l'invention contient du sorbitol. Pour une composition en acide glucuronique inférieure ou égale à 20 g/l de milieu, on additionne au milieu contenant du sorbitol, du 5-bromo-4-chloro-3-indolyl-β-D-glucopyranoside (esculine), en une quantité comprise entre 0,01 et 0,05 g par litre de milieu (et de préférence 0,025 g) ; l'esculine colore en mauve ou marron les éventuelles colonies de Serratia.

En présence de sorbitol, l'effet des inhibiteurs sur la croissance des bactéries des genres Proteus et Morganella est affaibli, et il convient alors d'augmenter la quantité des sels biliaires. De préférence, elle est comprise entre 1 et 5 g de désoxycholate de sodium, avantageusement entre 3 et 4 g par litre.

La préparation, la mise en oeuvre et les avantages d'un milieu de culture et de détection selon l'invention sont à présent illustrés par les exemples suivants.

### EXEMPLE 1 - Préparation d'un milieu selon l'invention

Une composition d'un milieu de l'invention est la suivante :
- bio-trypticase bioMérieux 5,00 g
- extrait de levure Difco 3,00 g
- NaCl 5,00 g
- rouge neutre 0,03 g
- désoxycholate de sodium 3,00 g
- vert brillant 0,01 g
- glucuronate de sodium 12,00 g
- 5-bromo-4-chloro-3-indolyl-β-D-galactopyranoside 0,10 g
- isopropyl-β-D-thiogalactoryranoside 0,01 g
- Agar 15,00 g
- pH 7,6

Les ingrédients ci-dessus, à l'exception du glucuronate de sodium, sont dissous dans l'eau distillée, en maintenant le milieu à ébullition pendant 1 minute, sous agitation.

La solution ainsi obtenue est autoclavée selon les procédés habituels de stérilisation (120°C pendant 15 mn par exemple).

Après autoclavage, la solution est refroidie et maintenue à 45°C. Le glucuronate de sodium est alors ajouté et dissous. Puis le milieu complet est réparti stérilement dans des boîtes de Pétri de diamètre 90 mm, à raison de 20 ml par boîte. La solidification est obtenue après retour à température ambiante. Les boîtes sont conservées à +4°C jusqu'à leur utilisation.

### EXEMPLE 2 - Détection sélective des Salmonella après culture sur un milieu selon l'invention

Les boîtes de Pétri préparées précédemment selon l'Exemple 1 sont ensemencées suivant le procédé habituel.

A 24 heures d'incubation à 37°C, les colonies formées sont examinées.

Selon la coloration des colonies, on est en mesure de déterminer les deux caractères biochimiques des bactéries, à savoir production ou non de β-galactosidase et fermentation ou non de l'acide glucuronique ou de son sel :
- colonies rouge brillant : elles sont spécifiques des bactéries qui ne produisent pas la β-galactosidase et qui fermentent l'acide glucuronique ou son sel
- colonies bleues : elles sont spécifiques des bactéries qui produisent la β-galactosidase et qui ne fermentent pas l'acide glucuronique ou son sel
- colonies violettes : elles sont spécifiques des bactéries qui produisent la β-galactosidase et qui fermentent l'acide glucuronique ou son sel
- colonies incolores : elles sont spécifiques des bactéries qui ne produisent pas la β-galactosidase et qui ne fermentent pas l'acide glucuronique ou son sel, à l'exception des bactéries du genre Serratia qui bien que productrices de la β-galactosidase restent incolores car l'enzyme ne s'exprime pas sur le milieu de l'invention.

Les colonies rouge brillant sont présumées être des Salmonella.

### EXEMPLE 3 - Préparation d'un milieu de l'invention en vue d'une détection sélective des Salmonella, par observation des colonies, après plus de 24 heures d'incubation.

Au-delà de 24 heures d'incubation à 37°C, sur le milieu de l'Exemple 1, dans les conditions de l'Exemple 2, les colonies colorées en rouge brillant tendent à devenir jaunes, à cause d'une réalcalinisation du pH, rendant la détection sélective des Salmonella plus difficile.

Pour empêcher le virage du rouge au jaune, une composition préférentielle d'un milieu de l'invention est la suivante :
- Pepticase 2 à 8 g, de préférence 3 g
- Thione 2 à 8 g, de préférence 3 g
- Extrait de viande 0 à 3 g, de préférence 3 g
- Extrait de levure 0 à 3 g, de préférence 2 g
- Vert brillant 0 à 0,01 g, de préférence 0,003 g
- Sels biliaires 1 à 5 g, de préférence 4 g
- Rouge neutre 0,025 g
- Tris 0,65 g
- Sorbitol 5 à 10 g, de préférence 8 g
- Sodium glucuronate 6 à 15 g, de préférence 12 g
- 5-Bromo-4-Chloro-3-Indolyl-β-D-Galactopyranoside : 0,17 g
- Isopropyl-β-D-Thiogalactopyranoside : 0,01 g
- 5-Bromo-4-Chloro-3-Indolyl-β-D-Glucopyranoside : 0,01 à 0,05 g, de préférence 0,025 g
- Agar 14 g
- pH 7,6

### EXEMPLE 4 - Mise en évidence de la sélectivité d'un milieu de détection selon l'invention

On considère les 32 souches suivantes :
3 souches d'Escherichia coli
2 souches de Serratia marcescens
3 souches de Citrobacter freundii
2 souches de Klebsiella pneumoniae
2 souches de Klebsiella oxytoca
4 souches de Salmonella enteritidis
3 souches de Salmonella typhi
3 souches de Proteus vulgaris
3 souches de Proteus mirabilis
3 souches de Staphylococcus aureus
2 souches de Streptococcus pyogenes
2 souches de Enterococcus faecalis.

Chaque souche a été cultivée en boîte de Pétri, sur le milieu de l'invention. Les boîtes ont été incubées à 36°C pendant 20 heures. Les colonies formées ont été alors examinées visuellement.

Parmi les 32 isolements, toutes les souches de Salmonella et seulement ces souches ont donné des colonies rouges, aspect caractéristique des Salmonella sur le milieu de l'invention.

Des colonies bleues, indiquant la présence d'une β-galactosidase et l'absence de fermentation de l'acide glucuronique, ont été observées pour une souche : celle de C. freundii.

Les 3 souches d'E. coli, les 4 souches de Klebsiella, et les 2 souches de C. freundii ont présenté des colonies violettes (association des 2 tests). Les 6 Proteus ont été inhibées partiellement, avec présence de colonies transparentes (absence de β-galactosidase et de fermentation du glucuronate).

Aucune des 7 souches de staphylocoques, streptocoques et entérocoques ne s'est développée sur le milieu.

Cet exemple montre clairement la spécificité de ce milieu : seules les colonies de Salmonella ont présenté une pigmentation rouge.

### EXEMPLE 5 - Comparaison de la sélectivité du milieu de détection des Salmonella selon l'invention et de celle d'un milieu couramment utilisé

La comparaison est effectuée entre le milieu décrit en Exemple 1 et le milieu gélosé S.S. commercialisé par Diagnostics Pasteur. Ce dernier, utilisé pour l'isolement sélectif des Salmonella et Shigella, est basé sur la détection des caractères biochimiques des bactéries liés à la fermentation des hydrates de carbone et à la production d'H₂S.

Chacune des 32 souches considérées dans l'Exemple 4 est cultivée à la fois sur le milieu selon l'invention et sur le milieu S.S.

A 24 heures d'incubation à 37°C, l'aspect des colonies est observé. Sont considérées comme suspectes les colonies :
- rouges sur le milieu selon l'invention (forte présomption de Salmonella)
- incolores (lactose négatif) ou à centre noir (production d'H₂S) sur milieu S.S.

| Nombre de souches ESPECE | MILIEU SELON L'INVENTION | GELOSE S.S |
|---|---|---|
| 3 E. coli | 3 x violet | 3 x rouge |
| 2 S. marcescens | 2 x incolore | 2 x incolore = SUSPICION |
| 3 C. freundii | 2 x violet ; 1 x bleu | 3 x centre noir = SUSPICION |
| 2 K. pneumoniae | 2 x violet | 2 x rouge |
| 2 K. oxytoca | 2 x violet | 2 x rouge |
| 4 S. enteritidis | 4 x rouge = SUSPICION | 4 x centre noir = SUSPICION |
| 3 S. typhi | 3 x rouge = SUSPICION | 3 x incolore = SUSPICION |
| 3 P. vulgaris | 3 x incolore | 3 x centre noir = SUSPICION |
| 3 P. mirabilis | 3 x incolore | 3 x centre noir = SUSPICION |
| 3 Staph. aureus | pas de croissance | pas de croissance |
| 2 Strep. pyogenes | pas de croissance | pas de croissance |
| 2 Ent. faecalis | pas de croissance | pas de croissance |

Sur le milieu selon l'invention, seules 7 souches ont donné des colonies rouges suspectes. Ce sont toutes des Salmonella.

Au contraire, sur milieu S.S, 18 souches ont été suspectées comme pouvant être des Salmonella, c'est-à-dire :
- les 7 Salmonella (colonies incolores avec centre noir)
- les 2 S. marcescens (colonies incolores)
- les 3 C. freundii (colonies avec centre noir)
- les 6 Proteus (colonies avec centre noir).

La sélectivité du milieu de détection selon l'invention permet de limiter considérablement le nombre des confirmations (7 contre 18 avec le milieu S.S) entraînant pour l'utilisation de ce milieu une économie de temps et d'argent par rapport à l'utilisation d'un milieu classique.

### EXEMPLE 6 - Seuil minimal de détection des Salmonella cultivées sur un milieu selon l'invention

L'essai a été réalisé sur les 4 souches suivantes, isolées sur gélose tryptricase-soja :
SE = Salmonella enteritidis,
ST = Salmonella typhi
EC = Escherichia coli
PA = Pseudomonas aeruginosa

Après culture de 24 heures à 37°C sur gélose trypticase-soja, pour chaque souche une suspension dans de l'eau physiologique est réalisée, et ajustée au point 1 de l'échelle de Mac Farland, soit environ 2 x 10⁸ bactéries par millilitre.

Ces suspensions sont ensuite diluées 10⁴ fois pour EC et PA, et 10⁴, 10⁵, 10⁶ fois pour SE et ST.

Les différentes suspensions diluées de Salmonella sont ensuite mélangées à volume égal avec les suspensions diluées de EC ou de PA. Des boîtes de Pétri contenant le milieu selon l'invention sont ensemencées avec chacun des mélanges obtenus, à l'aide d'une oese calibrée de 10 microlitres.

A 24 heures d'incubation à 37°C, le nombre de colonies de Salmonella et celui de non Salmonella sont comptés sur chaque boîte.

Les résultats sont rapportés dans le tableau ci-après.

| dilutions dans le mélange Salmonella / non Salmonella | Nombre de colonies Salmonella / non Salmonella | | | |
|---|---|---|---|---|
| | SE/EC | SE/PA | ST/EC | ST/PA |
| 10⁴/10⁴ | 125/97 | 91/118 | 142/160 | 83/136 |
| 10⁵/10⁴ | 15/117 | 11/85 | 10/121 | 7/99 |
| 10⁶/10⁴ | 1/110 | 2/94 | 1/133 | 0/105 |

Ces résultats mettent en évidence le seuil très bas de détection des Salmonella du milieu selon l'invention : il est en effet possible de repérer facilement jusqu'à une colonie de Salmonella (rouge) au milieu d'une centaine de colonies des bactéries n'appartenant pas à ce genre : EC détectées par une couleur violette et PA incolores. De plus, la pigmentation rouge des colonies de Salmonella est constante quelque soit leur nombre sur le milieu d'isolement.

## Revendications (Revendications pour l'(les) Etat(s) contractant(s) suivant(s): AT, BE, CH, IT, DE, DK, FR, GB, GR, IT, LU, NL, SE)

1. Procédé d'analyse bactériologique pour détecter sélectivement les Salmonella, selon lequel on met l'échantillon a analyser au contact d'un milieu d'identification comprenant un substrat nutritif métabolisé par lesdites bactéries, un composé chromogène ou fluorigène, susceptible d'être hydrolyse par l'enzyme β-galactosidase, caractérisé en ce que le milieu d'identification comprend en outre de l'acide glucuronique ou un sel de ce dernier, et un indicateur de pH.

2. Procédé selon la revendication 1, caractérisé en ce que le milieu d'identification comprend du glucuronate de sodium, dans une quantité comprise entre 1 et 30 g, pour un litre du milieu d'identification.

3. Procédé selon la revendication 1 ou 2, caractérisé en ce que le milieu d'identification comprend en outre au moins un sucre fermentescible par les Salmonella choisi parmi le mélibiose, le sorbitol, le dulcitol, le mannitol, le glucose et le glucuronate en quantité comprise entre 1 et 10 g par litre.

4. Procédé selon l'une quelconque des revendications 1 à 3, caractérisé en ce qu'on ajuste le pH à une valeur comprise entre 6 et 8.

5. Milieu de culture et de détection sélective de bactéries du genre Salmonella, comprenant un substrat nutritif métabolisé par lesdites bactéries, un composé chromogène ou fluorigène, susceptible d'être hydrolysé par l'enzyme β-galactosidase, caractérisé en ce que le milieu comprend en outre de l'acide glucuronique ou un sel de ce dernier, et un indicateur de pH.

6. Milieu selon la revendication 5, caractérisé en ce que la quantité d'acide glucuronique ou son sel est comprise entre 1 et 30 g par litre de milieu.

7. Milieu selon la revendication 5, caractérisé en ce qu'il comprend en outre au moins un sucre fermentescible par les Salmonella choisi parmi le mélibiose, le sorbitol, le dulcitol, le mannitol, le glucose et le glucuronate en quantité comprise entre 1 et 10 g par litre.

8. Milieu selon la revendication 7, caractérisé en ce que le milieu comprend du sorbitol en quantité comprise entre 5 et 10 g par litre. et de préférence 8 g par litre.

9. Milieu selon la revendication 7. caractérisé en ce qu'il comprend en outre du 5-bromo-4-chloro-3-indolyl-β-D-glucopyranoside dans une quantité comprise entre 0,01 g et 0,05 g pour un litre de milieu et de préférence 0.025g.

10. Milieu selon la revendication 5, caractérisé en ce que le composé chromogène ou fluorigène est choisi parmi les composés suivants, à savoir p-nitrophényl-β-D-galactopyranoside, 4-méthylumbelliferyl-β-D-galactopyranoside, 5-bromo-4-chloro-3-indolyl-β-D-galactopyranoside.

11. Milieu selon la revendication 10, caractérisé en ce que le composé chromogène est le 5-bromo-4-chloro-3-indolyl-β-D-galactopyranoside.

12. Milieu selon la revendication 11, caractérisé en ce que la quantité de 5-bromo-4-chloro-3-indolyl-β-D-galactopyranoside est comprise entre 0,01 et 1 g, pour un litre.

13. Milieu selon la revendication 5, caractérisé en ce qu'il comprend un inhibiteur de croissance des genres ou espèces autres que Salmonella, choisi dans le groupe comprenant le vert brillant, le désoxycholate de sodium, et un mélange de sels biliaires.

14. Milieu selon la revendication 13, caractérisé en ce qu'il comprend du vert brillant en quantité comprise entre 0 et 100 mg, et du désoxycholate de sodium en quantité comprise entre 0 et 10 g.

15. Milieu selon les revendications 13 et 14, caractérisé en ce que la quantité du désoxycholate de sodium est comprise entre 1 g et 5 g, et de préférence entre 3 et 4 g pour un litre de milieu.

16. Milieu selon la revendication 5, caractérisé en ce que le substrat nutritif comprend une peptone, un extrait de levure, du chlorure de sodium, dont les quantités sont comprises respectivement entre 1 et 20 g. 0 et 10 g et 0 et 10 g.

17. Milieu selon la revendication 5, caractérisé en ce que l'indicateur de pH est choisi parmi les composants suivants, à savoir rouge de phénol, bleu de bromothymol, pourpre de bromocrésol, rouge neutre.

18. Milieu selon la revendication 17, caractérisé en ce que l'indicateur de pH est le rouge neutre, dans une quantité comprise entre 5 et 100 mg.

19. Milieu selon la revendication 5, caractérisé en ce que le milieu comprend un inducteur de la β-galactosidase, à savoir le lactose ou l'isopropyl-β-D-thiogalactopyranoside.

20. Milieu selon la revendication 19, caractérisé en ce que l'inducteur de la β-galactosidase est l'isopropyl-β-D-thiogalactopyranoside, dans une quantité comprise entre 0 et 100 mg pour un litre de milieu.

21. Milieu selon l'une quelconque des revendications 5 à 20, caractérise en ce que son pH est ajusté à une valeur comprise entre 6 et 8.

22. Milieu selon la revendication 5, caractérisé en ce que ledit milieu se présente sous forme solide gélosée.

23. Dispositif de caractérisation bactériologique, comprenant un conditionnement d'un milieu selon l'une quelconque des revendications 5 à 22.

## Revendications (Revendications pour l'(les) Etat(s) contractant(s) suivant(s): ES)

1. Procédé d'analyse bactériologique pour détecter sélectivement les *Salmonella,* selon lequel on met l'échantillon à analyser au contact d'un milieu d'identification comprenant un substrat nutritif métabolisé par lesdites bactéries, un composé chromogène ou fluorigène, susceptible d'être hydrolysé par l'enzyme β-galactosidase, caractérisé en ce que le milieu d'identification comprend en outre de l'acide glucuronique ou un sel de ce dernier, et un indicateur de pH.

2. Procédé selon la revendication 1, caractérisé en ce que le milieu d'identification comprend du glucuronate de sodium, dans une quantité comprise entre 1 et 30 g, pour un litre du milieu d'identification.

3. Procédé selon la revendication 1 ou 2, caractérisé en ce que le milieu d'identification comprend en outre au moins un sucre fermentescible par les *Salmonella* choisi parmi le mélibiose, le sorbitol, le dulcitol, le mannitol, le glucose et le glucuronate en quantité comprise entre 1 et 10 g par litre.

4. Procédé selon la revendication 3, caractérisé en ce que le milieu comprend du sorbitol en quantité comprise entre 5 et 10 g par litre, et de préférence 8 g par litre.

5. Procédé selon la revendication 3, caractérisé en ce que le milieu comprend en outre du 5-bromo-4-chloro-3-indolyl-β-D-glucopyranoside dans une quantité comprise entre 0,01 g et 0,05 g pour un litre de milieu et de préférence 0,025 g.

6. Procédé selon la revendication 1, caractérisé en ce que le composé chromogène ou fluorigène est choisi parmi les composés suivants, à savoir p-nitrophényl-β-D-galactopyranoside, 4-méthylumbelliferyl-β-D-galactopyranoside, 5-bromo-4-chloro-3-indolyl-β-D-galactopyranoside.

7. Procédé selon la revendication 6, caractérisé en ce que le composé chromogène est le 5-bromo-4-chloro-3-indolyl-β-D-galactopyranoside.

8. Procédé selon la revendication 7, caractérisé en ce que la quantité de 5-bromo-4-chloro-3-indolyl-β-D-galactopyranoside est comprise entre 0,01 et 1 g pour un litre.

9. Procédé selon la revendication 1, caractérisé en ce que le milieu comprend un inhibiteur de croissance des genres ou espèces autres que *Salmonella,* choisi dans le groupe comprenant le vert brillant, le désoxycholate de sodium, et un mélange de sels biliaires.

10. Procédé selon la revendication 9, caractérisé en ce que le milieu comprend du vert brillant en quantité entre 0 et 100 mg, et du désoxycholate de sodium en quantité comprise entre 0 et 10 g.

11. Procédé selon les revendications 9 et 10, caractérisé en ce que la quantité du désoxycholate de sodium est comprise entre 1 g et 5 g, et de préférence entre 3 et 4 g pour un litre de milieu.

12. Procédé selon la revendication 1, caractérisé en ce que le substrat nutritif comprend une peptone, un extrait de levure, du chlorure de sodium, dont les quantités sont comprises respectivement entre 1 et 20 g, 0 et 10 g et 0 et 10 g.

13. Procédé selon la revendication 1, caractérisé en ce que l'indicateur de pH est choisi parmi les composants suivants, à savoir rouge de phénol, bleu de bromothymol, pourpre de bromocrésol, rouge neutre.

14. Procédé selon la revendication 13, caractérisé en ce que l'indicateur de pH est le rouge neutre, dans une quantité comprise entre 5 et 100 mg.

15. Procédé selon la revendication 1, caractérisé en ce que le milieu comprend un inducteur de la β-galactosidase, à savoir le lactose ou l'isopropyl-β-D-thiogalactopyranoside.

16. Procédé selon la revendication 15, caractérisé en ce que l'inducteur de la β-galactosidase est l'isopropyl-β-D-thiogalactopyranoside, dans une quantité comprise entre 0 et 100 mg pour un litre de milieu.

17. Procédé selon l'une quelconque des revendications 1 à 16, caractérisé en ce que le pH du milieu est ajusté à une valeur comprise entre 6 et 8.

18. Procédé selon la revendication 1, caractérisé en ce que ledit milieu se présente sous forme solide gélosée.

## Patentansprüche (Patentansprüche für folgende(n) Vertragsstaat(en): AT, BE, CH, IT, DE, DK, FR, GB, GR, IT, LU, NL, SE)

1. Bakteriologisches Analyseverfahren zum selektiven Nachweis von Salmonella, bei dem man die zu analysierende Probe mit einem Nachweismedium in Kontakt bringt, das ein Nährsubstrat, welches durch die besagten Bakterien metabolisiert wird, und eine chromogene oder fluorogene Verbindung enthält, welche durch das Enzym β-Galctosidase hydrolysiert werden kann, dadurch gekennzeichnet, daß das Nachweismedium außerdem Glucuronsäure oder eines ihrer Salze und einen pH-Indikator enthält.

2. Verfahren nach Anspruch 1, dadurch gekennzeichnet,daß das Nachweismedium Natriumglucuronat in einer Menge zwischen 1 und 30 g pro Liter Nachweismedium enthält.

3. Verfahren nach Anspruch 1 oder 2, dadurch gekennzeichnet, daß das Nachweismedium außerdem mindestens einen durch Salmonella fermentierbaren Zucker, ausgewählt aus Melibiose, Sorbitol, Dulcit, Mannitol, Glucose und Glucuronat, in einer Menge zwischen 1 und 10 g pro Liter enthält.

4. Verfahren nach einem der Ansprüche 1 bis 3, dadurch gekennzeichnet, daß man den pH auf einen Wert zwischen 6 und 8 einstellt.

5. Medium zur Kultivierung und zum selektiven Nachweis von Bakterien der Gattung Salmonella, das ein Nährsubstrat, welches durch die besagten Bakterien metabolisiert wird, und eine chromogene oder fluorogene Verbindung enthält, welche durch das Enzym β-Galctosidase hydrolysiert werden kann, dadurch gekennzeichnet, daß das Nachweismedium außerdem Glucuronsäure oder eines ihrer Salze und einen pH-Indikator enthält.

6. Medium nach Anspruch 5, dadurch gekennzeichnet, daß die Menge an Glucuronsäure oder deren Salz zwischen 1 und 30 g pro Liter Medium liegt.

7. Medium nach Anspruch 5, dadurch gekennzeichnet, daß es außerdem mindestens einen durch die Salmonella fermentierbaren Zucker, ausgewählt aus Melibiose, Sorbitol, Dulcit, Mannitol, Glucose und Glucuronat, in einer Menge zwischen 1 und 10 g pro Liter enthält.

8. Medium nach Anspruch 7, dadurch gekennzeichnet, daß das Medium Sorbitol in einer Menge zwischen 5 und 10 g pro Liter, vorzugsweise mit 8 g pro Liter enthält.

9. Medium nach Anspruch 7, dadurch gekennzeichnet, daß es außerdem 5-Brom-4-chlor-3-indolyl-β-D-glukopyranosid in einer Menge zwischen 0,01 g und 0,05 g, vorzugsweise mit 0,025 g, pro Liter Medium enthält.

10. Medium nach Anspruch 5, dadurch gekennzeichnet, daß die chromogene oder fluorogene Verbindung unter den folgenden Verbindungen ausgewählt ist, nämlich p-Nitrophenyl-β-D-galaktopyranosid, 4-Methylumbelliferyl-β-D-galaktopyranosid, 5-Brom-4-chlor-3-indolyl-β-D-galaktopyranosid.

11. Medium nach Anspruch 10, dadurch gekennzeichnet, daß die chromogene Verbindung 5-Brom-4-chlor-3-indolyl-β-D-galaktopyranosid ist.

12. Medium nach Anspruch 11 , dadurch gekennzeichnet, daß die Menge an 5-Brom-4-chlor-3-indolyl-β-D-galaktopyranosid zwischen 0,01 und 1 g pro Liter liegt.

13. Medium nach Anspruch 5, dadurch gekennzeichnet, daß es einen Wachstumshemmer für andere Gattungen oder Arten als Salmonella enthält, ausgewählt aus der Gruppe bestehend aus Brilliantgrün, Natriumdesoxycholat und einer Mischung von Salzen der Gallensäuren.

14. Medium nach Anspruch 13, dadurch gekennzeichnet, daß es Brilliantgrün in einer Menge zwischen 0 und 100 mg und Natriumdesoxycholat in einer Menge zwischen 0 und 10 g enthält.

15. Medium nach Anspruch 13 und 14, dadurch gekennzeichnet, daß die Menge an Natriumdesoxycholat zwischen 1 g und 5 g, vorzugsweise zwischen 3g und 4 g pro Liter Medium liegt.

16. Medium nach Anspruch 5, dadurch gekennzeichnet, daß das Nährsubstrat ein Pepton, einen Hefeextrakt und Kochsalz enthält, deren Mengen jeweils zwischen 1 und 20 g bzw. 0 und 10 g bzw. 0 und 10 g liegen.

17. Medium nach Anspruch 5, dadurch gekennzeichnet, daß der pH-Indikator unter den folgenden Verbindungen, nämlich Phenolrot, Bromthymolblau, Bromkresolpurpur, Neutralrot, ausgewählt ist.

18. Medium nach Anspruch 17, dadurch gekennzeichnet, daß der pH-Indikator Neutralrot, in einer Menge zwischen 5 und 100 mg, ist.

19. Medium nach Anspruch 5, dadurch gekennzeichnet, daß das Medium einen Induktor für die β-Galaktosidase, nämlich Laktose oder Isopropyl-β-D-thiogalaktopyranosid enthält.

20. Medium nach Anspruch 19, dadurch gekennzeichnet, daß Induktor für die β-Galaktosidase Isopropyl-β-D-thiogalaktopyranosid, in einer Menge zwischen 0 und 100 mg pro Liter Medium, ist.

21. Medium nach einem der Ansprüche 5 bis 20 , dadurch gekennzeichnet, daß sein pH auf einen Wert zwischen 6 und 8 eingestellt ist.

22. Medium nach Anspruch 5 , dadurch gekennzeichnet, daß das besagte Medium in fester Agarform vorliegt.

23. Vorrichtung zur bakteriologischen Charakterisierung, die ein nach einem der Ansprüche 5 bis 22 aufbereitetes Medium enthält.

## Patentansprüche (Patentansprüche für folgende(n) Vertragsstaat(en): ES)

1. Bakteriologisches Analyseverfahren zum selektiven Nachweis von Salmonella, bei dem man die zu analysierende Probe mit einem Nachweismedium in Kontakt bringt, das ein Nährsubstrat, welches durch die besagten Bakterien metabolisiert wird, und eine chromogene oder fluorogene Verbindung enthält, welche durch das Enzym β-Galctosidase hydrolysiert werden kann, dadurch gekennzeichnet, daß das Nachweismedium außerdem Glucuronsäure oder eines ihrer Salze und einen pH-Indikator enthält.

2. Verfahren nach Anspruch 1, dadurch gekennzeichnet, daß das Nachweismedium Natriumglucuronat in einer Menge zwischen 1 und 30 g pro Liter Nachweismedium enthält.

3. Verfahren nach Anspruch 1 oder 2, dadurch gekennzeichnet, daß das Nachweismedium außerdem mindestens einen durch Salmonella fermentierbaren Zucker, ausgewählt aus Melibiose, Sorbitol, Dulcit, Mannitol, Glucose und Glucuronat, in einer Menge zwischen 1 und 10 g pro Liter enthält.

4. Verfahren nach Anspruch 3, dadurch gekennzeichnet, daß das Medium Sorbitol in einer Menge zwischen 5 und 10 g pro Liter, vorzugsweise mit 8 g pro Liter enthält.

5. Verfahren nach Anspruch 3, dadurch gekennzeichnet, daß das Medium außerdem 5-Brom-4-chlor-3-indolyl-β-D-glukopyranosid in einer Menge zwischen 0,01 g und 0,05 g, vorzugsweise mit 0,025 g pro Liter Medium enthält.

6. Verfahren nach Anspruch 1, dadurch gekennzeichnet, daß die chromogene oder fluorogene Verbindung unter den folgenden Verbindungen ausgewählt ist, nämlich p-Nitrophenyl-β-D-galaktopyranosid, 4 Methylumbelliferyl-β-D-galaktopyranosid, 5-Brom-4-chlor-3-indolyl-β-D-galaktopyranosid.

7. Verfahren nach Anspruch 6, dadurch gekennzeichnet, daß die chromogene Verbindung 5-Brom-4-chlor-3-indolyl-β-D-galaktopyranosid ist.

8. Verfahren nach Anspruch 7, dadurch gekennzeichnet, daß die Menge an 5-Brom-4-chlor-3-indolyl-β-D-galaktopyranosid zwischen 0,01 und 1 g pro Liter liegt.

9. Verfahren nach Anspruch 1, dadurch gekennzeichnet, daß das Medium einen Wachstumshemmer für andere Gattungen oder Arten als Salmonella enthält, der ausgewählt ist aus der Gruppe bestehend aus Brilliantgrün, Natriumdesoxycholat und einer Mischung von Salzen der Gallensäuren.

10. Verfahren nach Anspruch 9, dadurch gekennzeichnet, daß das Medium Brilliantgrün in einer Menge zwischen 0 und 100 mg und Natriumdesoxycholat in einer Menge zwischen 0 und 10 g enthält.

11. Verfahren nach Anspruch 9 und 10, dadurch gekennzeichnet, daß die Menge Natriumdesoxycholat zwischen 1 g und 5 g, vorzugsweise zwischen 3g und 4 g pro Liter Medium liegt.

12. Verfahren nach Anspruch 1, dadurch gekennzeichnet, daß das Nährsubstrat ein Pepton, einen Hefeextrakt und Kochsalz enthält, deren Mengen jeweils zwischen 1 und 20 g bzw. 0 und 10 g bzw. 0 und 10 g liegen.

13. Verfahren nach Anspruch 1, dadurch gekennzeichnet, daß der pH-Indikator unter den folgenden Verbindungen, nämlich Phenolrot, Bromthymolblau, Bromkresolpurpur, Neutralrot, ausgewählt ist.

14. Verfahren nach Anspruch 13, dadurch gekennzeichnet, daß der pH-Indikator Neutralrot, in einer Menge zwischen 5 und 100 mg, ist.

15. Verfahren nach Anspruch 1, dadurch gekennzeichnet, daß das Medium einen Induktor für die β-Galaktosidase, nämlich Laktose oder Isopropyl-β-D-thiogalakto-pyranosid enthält.

16. Verfahren nach Anspruch 15, dadurch gekennzeichnet, daß der Induktor für die β-Galaktosidase Isopropyl-β-D-thiogalaktopyranosid, in einer Menge zwischen 0 und 100 mg pro Liter Medium, ist.

17. Verfahren nach einem der Ansprüche 1 bis 16, dadurch gekennzeichnet, daß der pH des Mediums auf einen Wert zwischen 6 und 8 eingestellt ist.

18. Verfahren nach Anspruch 1, dadurch gekennzeichnet, daß das besagte Medium in fester Agarform vorliegt.

## Claims (Claims for the following Contracting State(s): AT, BE, CH, IT, DE, DK, FR, GB, GR, IT, LU, NL, SE)

1. Method of bacteriological analysis for the selective detection of Salmonella, according to which the analytical sample is placed in contact with an identification medium comprising a nutrient which is metabolised by the said bacteria and a chromogenic or fluorigenic compound capable of being hydrolysed by the enzyme β-galactosidase, characterised in that the identification medium comprises, in addition, glucuronic acid or a salt thereof and a pH indicator.

2. Method according to Claim 1, characterised in that the identification medium comprises sodium glucuronate in an amount of between 1 and 30 g per one litre of identification medium.

3. Method according to Claim 1 or 2, characterised in that the identification medium comprises, in addition, at least one sugar which can be fermented by Salmonella, chosen from melibiose, sorbitol, dulcitol, mannitol, glucose and glucuronate in an amount of between 1 and 10 g per litre.

4. Method according to any one of Claims 1 to 3, characterised in that the pH is adjusted to a value of between 6 and 8.

5. Medium for the culture and selective detection of bacteria of the Salmonella genus, comprising a nutrient which is metabolised by the said bacteria and a chromogenic or fluorigenic compound capable of being hydrolysed by the enzyme β-galactosidase, characterised in that the medium comprises, in addition, glucuronic acid or a salt thereof and a pH indicator.

6. Medium according to Claim 5, characterised in that the amount of glucuronic acid or its salt is between 1 and 30 g per litre of medium.

7. Medium according to Claim 5, characterised in that it comprises, in addition, at least one sugar which can be fermented by Salmonella, chosen from melibiose, sorbitol, dulcitol, mannitol, glucose and glucuronate, in an amount of between 1 and 10 g per litre.

8. Medium according to Claim 7, characterised in that the medium comprises sorbitol in an amount of between 5 and 10 g per litre, and preferably 8 g per litre.

9. Medium according to Claim 7, characterised in that it comprises, in addition, 5-bromo-4-chloro-3-indolyl β-D-glucopyranoside in an amount of between 0.01 and 0.05 g per litre of medium, preferably 0.025 g.

10. Medium according to Claim 5, characterised in that the chromogenic or fluorigenic compound is chosen from the following compounds, namely p-nitrophenyl β-D-galactopyranoside, 4-methylumbelliferyl β-D-galactopyranoside and 5-bromo-4-chloro-3-indolyl β-D-galactopyranoside.

11. Medium according to Claim 10, characterised in that the chromogenic compound is 5-bromo-4-chloro-3-indolyl β-D-galactopyranoside.

12. Medium according to Claim 11, characterised in that the amount of 5-bromo-4-chloro-3-indolyl β-D-galactopyranoside is between 0.01 and 1 g per litre.

13. Medium according to Claim 5, characterised in that it comprises an inhibitor of the growth of genera or species other than Salmonella, chosen from the group comprising brilliant green, sodium deoxycholate and a mixture of bile salts.

14. Medium according to Claim 13, characterised in that it comprises brilliant green in an amount of between 0 and 100 mg, and sodium deoxycholate in an amount of between 0 and 10 g.

15. Medium according to Claims 13 and 14, characterised in that the amount of sodium deoxycholate is between 1 g and 5 g, preferably between 3 and 4 g per litre of medium.

16. Medium according to Claim 5, characterised in that the nutrient comprises a peptone, a yeast extract and sodium chloride, the amounts of which are between 1 and 20 g, 0 and 10 g and 0 and 10 g, respectively.

17. Medium according to Claim 5, characterised in that the pH indicator is chosen from the following compounds, namely phenol red, bromothymol blue, bromocresol purple and neutral red.

18. Medium according to Claim 17, characterised in that the pH indicator is neutral red, in an amount of between 5 and 100 mg.

19. Medium according to Claim 5, characterised in that the medium comprises a β-galactosidase inducer, namely lactose or isopropyl β-D-thiogalactopyranoside.

20. Medium according to Claim 19, characterised in that the B-galactosidase inducer is isopropyl β-D-thiogalactopyranoside, in an amount of between 0 and 100 mg per litre of medium.

21. Medium according to any one of Claims 5 to 20, characterised in that its pH is adjusted to a value of between 6 and 8.

22. Medium according to Claim 5, characterised in that the said medium is provided in the form of solid agar.

23. Device for bacteriological characterisation, comprising a package of a medium according to any one of Claims 5 to 22.

## Claims (Claims for the following Contracting State(s): ES)

1. Method of bacteriological analysis for the selective detection of Salmonella, according to which the analytical sample is placed in contact with an identification medium comprising a nutrient which is metabolised by the said bacteria and a chromogenic or fluorigenic compound capable of being hydrolysed by the enzyme β-galactosidase, characterised in that the identification medium comprises, in addition, glucuronic acid or a salt thereof and a pH indicator.

2. Method according to Claim 1, characterised in that the identification medium comprises sodium glucuronate in an amount of between 1 and 30 g per one litre of identification medium.

3. Method according to Claim 1 or 2, characterised in that the identification medium comprises, in addition, at least one sugar which can be fermented by Salmonella, chosen from melibiose, sorbitol, dulcitol, mannitol, glucose and glucuronate in an amount of between 1 and 10 g per litre.

4. Method according to Claim 3, characterised in that the medium comprises sorbitol in an amount of between 5 and 10 g per litre, and preferably 8 g per litre.

5. Method according to Claim 3, characterised in that the medium comprises, in addition, 5-bromo-4-chloro3-indolyl β-D-glucopyranoside in an amount of between 0.01 and 0.05 g per litre of medium, preferably 0.025 g.

6. Method according to Claim 1, characterised in that the chromogenic or fluorigenic compound is chosen from the following compounds, namely p-nitrophenyl β-D-galactopyranoside, 4-methylumbelliferyl β-D-galactopyranoside and 5-bromo-4-chloro-3-indolyl β-D-galactopyranoside.

7. Method according to Claim 6, characterised in that the chromogenic compound is 5-bromo-4-chloro-3-indolyl β-D-galactopyranoside.

8. Method according to Claim 7, characterised in that the amount of 5-bromo-4-chloro-3-indolyl β-D-galactopyranoside is between 0.01 and 1 g per litre.

9. Method according to Claim 1, characterised in that the medium comprises an inhibitor of the growth of genera or species other than Salmonella, chosen from the group comprising brilliant green, sodium deoxycholate and a mixture of bile salts.

10. Method according to Claim 9, characterised in that the medium comprises brilliant green in an amount of between 0 and 100 mg, and sodium deoxycholate in an amount of between 0 and 10 g.

11. Method according to Claims 9 and 10, characterised in that the amount of sodium deoxycholate is between 1 g and 5 g, preferably between 3 and 4 g per litre of medium.

12. Method according to Claim 1, characterised in that the nutrient comprises a peptone, a yeast extract and sodium chloride, the amounts of which are between 1 and 20 g, 0 and 10 g and 0 and 10 g, respectively.

13. Method according to Claim 1, characterised in that the pH indicator is chosen from the following compounds, namely phenol red, bromothymol blue, bromocresol purple and neutral red.

14. Method according to Claim 13, characterised in that the pH indicator is neutral red, in an amount of between 5 and 100 mg.

15. Method according to Claim 1, characterised in that the medium comprises a β-galactosidase inducer, namely lactose or isopropyl β-D-thiogalactopyranoside.

16. Method according to Claim 15, characterised in that the B-galactosidase inducer is isopropyl β-D-thiogalactopyranoside, in an amount of between 0 and 100 mg per litre of medium.

17. Method according to any one of Claims 1 to 16, characterised in that its pH is adjusted to a value of between 6 and 8.

18. Method according to Claim 1, characterised in that the said medium is provided in the form of solid agar.
